# EUROPEAN PATENT APPLICATION

(11) **EP 2 181 999 A1**
(43) Date of publication of application: **05.05.2010**
(21) Application number: 08019162.0
(22) Date of filing: 03.11.2008
(51) Int. Cl.: C07D 498/22

(54) **Method of manufacturing ruboxistarin**

(71) Applicant: Zentiva, k.s., 102 37 Praha 10 (CZ)
(72) Inventor: Kral, Vladimir, 120 00 Praha 2 (CZ); Jirman, Josef, 100 00 Praha 10 (CZ); Flubacher, Dietmar, 79189 Bad Krozingen (DE)
(74) Representative: Jirotkova, Ivana

(57) **Abstract**

Method of manufacturing ruboxistaurin, comprising the steps of preparation of L-2-deoxyribose 1-cyano-3,4-dibenzoate, followed by deprotection to 1-cyano L-2-deoxyribose, oxidative cleaving to (S)-4-hydroxy-2-(2-hydroxy-ethoxy)-butyronitrile and transformation to the methanesulfonic acid (S)-3-cyano-3-(2-methanesulfonyloxyethoxy)-propyl ester, then coupling with 1-substituted-3,4-bis(3-indolyl)maleimide leading to 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-substituted-9(S)-cyano, followed by methylation of the amino group and deprotection forming 5,21:12,17-dimetheno-9H-dibenzo[e,k]furo[3,4-h][1,4,13]oxadiazacyclohexadecine-18,20-dione, 6,7,10,11-tetrahydro-9-[(dimethylamino)methyl]-, (S)- (9CI), and the final step consisting of imido preparation to form ruboxistaurin.

## Description

### Technical Field:

The present invention relates to a method of manufacturing ruboxistaurin.

### State of the art:

Ruboxistaurin **1** displayed in Scheme 1 was first described by Eli Lilly in EP0657458¹ as a potent protein kinase C inhibitor. It is a synthetic analogue derived from staurosporin **2**, which is a natural product

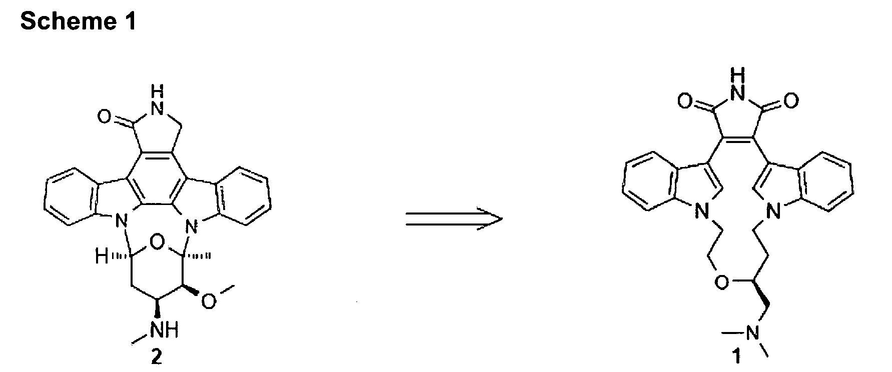

The lactam moiety of **2** is replaced by a symmetric imide ring resulting in a symmetric aglycone ring system. The glycone is replaced by an open chain linker between the indole nitrogens of the aglycone moiety.

Protein kinase C (PKC) consists of a family of closely related enzymes that function as serine/threonine kinases. Protein kinase C plays an important role in cell-cell signalling, gene expression, and in the control of cell differentiation and growth. At present, there are currently at least ten known isozymes of PKC that differ in their tissue distribution, enzymatic specificity, and regulation². Protein kinase C isozymes are single polypeptide chains ranging from 592 to 737 amino acids in length. The isozymes contain a regulatory domain and a catalytic domain connected by a linker peptide. The regulatory and catalytic domains can be further subdivided into constant and variable regions. The catalytic domain of protein kinase C is very similar to that seen in other protein kinases while the regulatory domain is unique to the PKC isozymes. The PKC isozymes demonstrate between 40-80% homology at the amino acid level among the group. However, the homology of a single isozyme between different species is generally greater than 97%. Protein kinase C is a membrane-associated enzyme that is allosterically regulated by a number of factors, including membrane phospholipids, calcium, and certain membrane lipids such as diacylglycerols that are liberated in response to the activities of phospholipases^{2,3}. The protein kinase C isozymes, alpha, beta-1, beta-2 and gamma, require membrane phospholipid, calcium and diacylglycerol/phorbol esters for full activation. The delta, epsilon, eta, and theta forms of PKC are calcium-independent in their mode of activation. The zeta and lambda forms of PKC are independent of both calcium and diacylglycerol and are believed to require only membrane phospholipid for their activation. Only one or two of the protein kinase C isozymes may be involved in a given disease state. For example, the elevated blood glucose levels found in diabetes lead to an isozyme-specific elevation of the beta-2 isozyme in vascular tissues⁴. A diabetes-linked elevation of the beta isozyme in human platelets has been correlated with their altered response to agonists⁵. The human vitamin D receptor has been shown to be selectively phosphorylated by protein kinase C beta. This phosphorylation has been linked to alterations in the functioning of the receptor⁶. In addition, recent work has shown that the beta-2 isozyme is responsible for erythroleukemia cell proliferation while the alpha isozyme is involved in megakaryocyte differentiation in these same cells⁷. The ubiquitous nature of the protein kinase C isozymes and their important roles in physiology provide incentives to produce highly selective PKC inhibitors. Given the evidence demonstrating linkage of certain isozymes to disease states, it is reasonable to assume that inhibitory compounds that are selective to one or two protein kinase C isozymes relative to the other PKC isozymes and other protein kinases are superior therapeutic agents. Such compounds should demonstrate greater efficacy and lower toxicity by virtue of their specificity. The microbial indolocarbazole, Staurosporin, is a potent inhibitor of protein kinase C that interacts with the catalytic domain of the enzyme⁸. However, the therapeutic usefulness of this molecule and closely related compounds is limited by the lack of specificity for protein kinase C over other protein kinases⁹. This lack of selectivity results in unacceptable toxicity in this class of molecules. An additional class of compounds related to Staurosporin, the bisindolemaleimides, has been the focus of recent work¹⁰. Some of these compounds have demonstrated selectivity for protein kinase C over other protein kinases. EP0410389¹¹ discloses indolocarbazol analogues and their use in the treatment of thromboses, arterioscleroses, hypertension, and for treating inflammation, allergies, cancer, and certain degenerative damage of the central nervous system as well as diseases of the immune system. EP0508792¹² discloses bisindolemaleimide analogues and their use as anti-tumor and antipsoriasis agents. WO94/07895¹³ discloses bisindolemaleimide derivatives and their use as anti-tumor and anti-inflammatory agents. Although compounds that demonstrate specificity to protein kinase C have been discovered, very little is known regarding isozyme selectivity. For example, analysis of the isozyme selectivity of Staurosporin, shows little isozyme selectivity with the exception of poor inhibition of the zeta isozyme relative to the other isozymes¹⁴. Studies of the PKC-selective compound, 3-[1-(3-dimethylaminopropyl)-indol-3-yl]-4-(1H-indol-3-yl)-1H-pyrrole-2,5-dione, suggest a slight selectivity for the calcium dependent isozymes¹⁵. Subsequent studies of this compound observed no difference, or possibly slight selectivity, for alpha over beta-5 1 and beta-2 isozymes¹⁶. Therefore, despite years of research and the identification of classes of compounds that inhibit protein kinase C versus other protein kinases, there remains a need for therapeutically effective isozyme-selective inhibitors.
Ruboxistaurin is disclosed in EP0657458¹ and is claimed to be a novel and potent protein kinase C inhibitor. The compounds disclosed herein are selective to protein kinase C over other kinases and are highly isozyme-selective. As selective inhibitors the compounds are useful in treating conditions associated with diabetes mellitus and its complications, ischemia, inflammation, central nervous system disorders, cardiovascular disease, dermatological disease and cancer.

### Retro synthetic Analysis of Ruboxistaurin

Different strategies for macrocyclization yielding Ruboxistaurin or precursor A retro synthetic analysis is displayed in Scheme 2 revealing following possibilities for synthetic strategies:
A) Cyclization by using an N-Indolyl substituted acetamide with an unsubstituted gyloxalate
B) Cyclization by using an N-Indolyl substituted Gyloxalate with an unsubstituted Acetamide
C) Cyclization of a functionalized bis-indole
D) Cyclization of Bis-indolylmaleimide with a functionalized linker.

A detailed study focusing on the methods A), B) and C is described in a paper by M. M. Faul and Ch. A. Krumrich¹⁷.

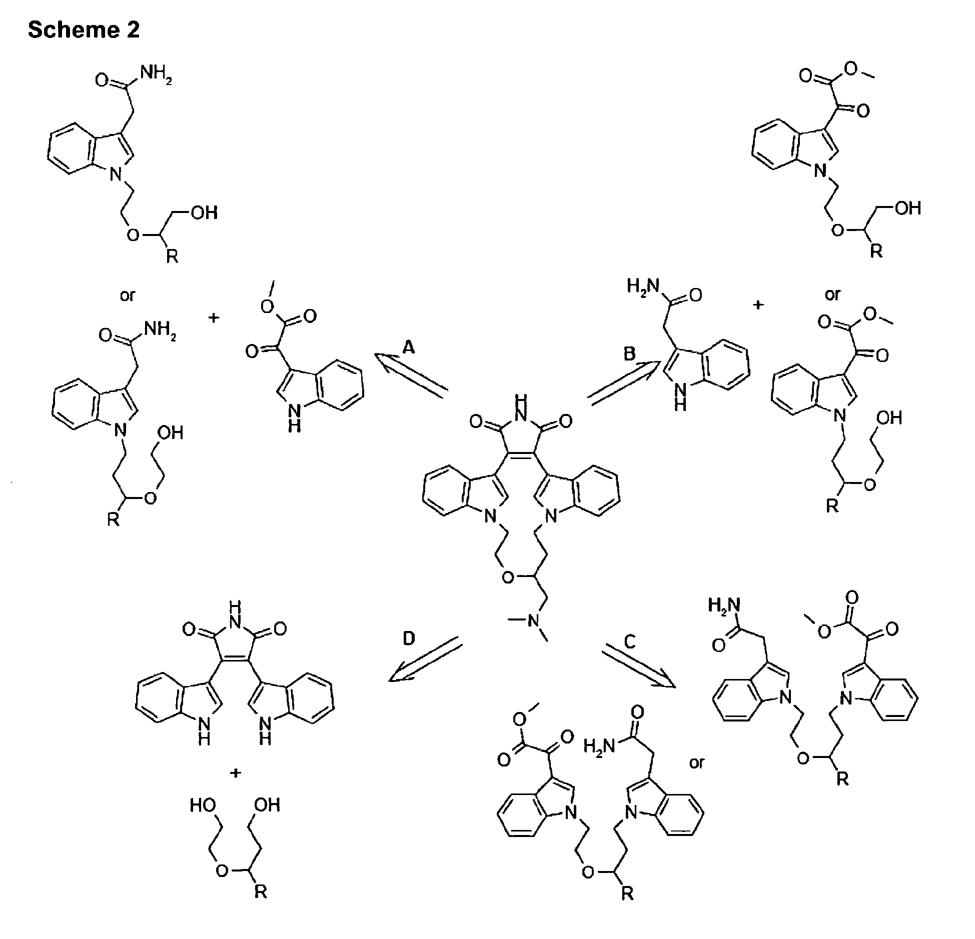

Different strategies for preparation of 2-(2-Hydroxy-ethoxy)-butane-1,4-diol linker of Ruboxistaurin. Up to now we can identify three general approaches for the preparation of the linker moiety of Ruboxistaurin (Scheme 3). All of these strategies use an intermediate with a protected hydroxyl-group serving as precursor for the dimethylamino group of Ruboxistaurin. This intermediate is prepared by three distinct starting materials:
F) Malic acid
G) (R)-3,6-Dihydro-2H-pyran-2-carboxylic acid
H) R-Glycidol

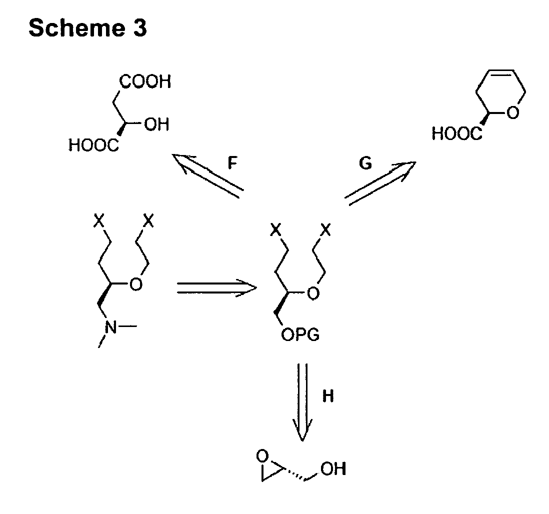

Sequence F using malic acid or derivatives thereof are described in JP1995238044¹⁸ EP0657458¹, CA2137203¹⁹, US5624949²⁰, JP2004269543²¹ and papers by Jirousek et al.²² and M. M. Faul¹⁷.The second route (G) is described by J. C. Camille²³ and W002059342²⁴. The Glycerol-approach (H) is the most widely investigated approach and is described in several patents²⁵.

### Synthetic Approaches towards Ruboxistaurin via Route D

There are several approaches described for this synthetic strategy28. The route using (S)-malic dimethyl ester as starting material is displayed in Scheme 4. The bis-mesylate used in the macrolactonization is prepared via a 6-step sequence with an overall yield of 40 %. A different synthetic path is pictured in Scheme 5 using R-gylcidol as starting material. Several bis-halides and the bis-mesylate are prepared by a 5-6 step sequence with yields between 47 % and 62 %. Even though the overall yields are promising, there are some disadvantages as well. The first route required the use of allyl trichloroacetimidate to introduce the allyl moiety because apparently under basic conditions a silyl-migration occurred. Furthermore all intermediates were oils requiring chromatographic purification. According to the authors the second approach gave the product in good yields but due to the limited enantiomeric purity of the starting material. By introducing a trityl group they were able to obtain enriched ( from 89 %ee to 94 %ee) protected R-glycidol. However, the enantiomeric purity of the final product was still 94 % ee, which was not of sufficient quality for clinical trials. Besides both routes involve use of ozone which as previously mentioned is a challenging.

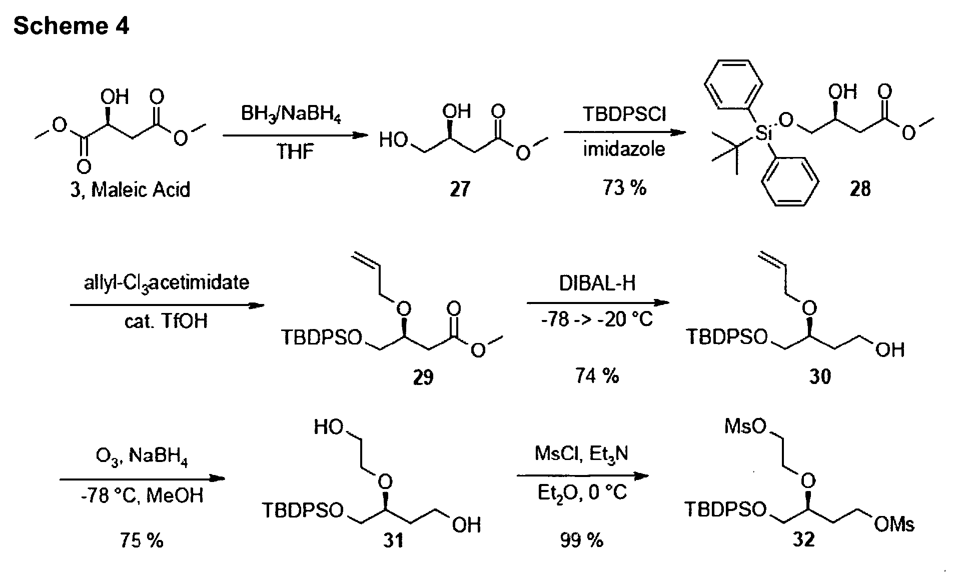

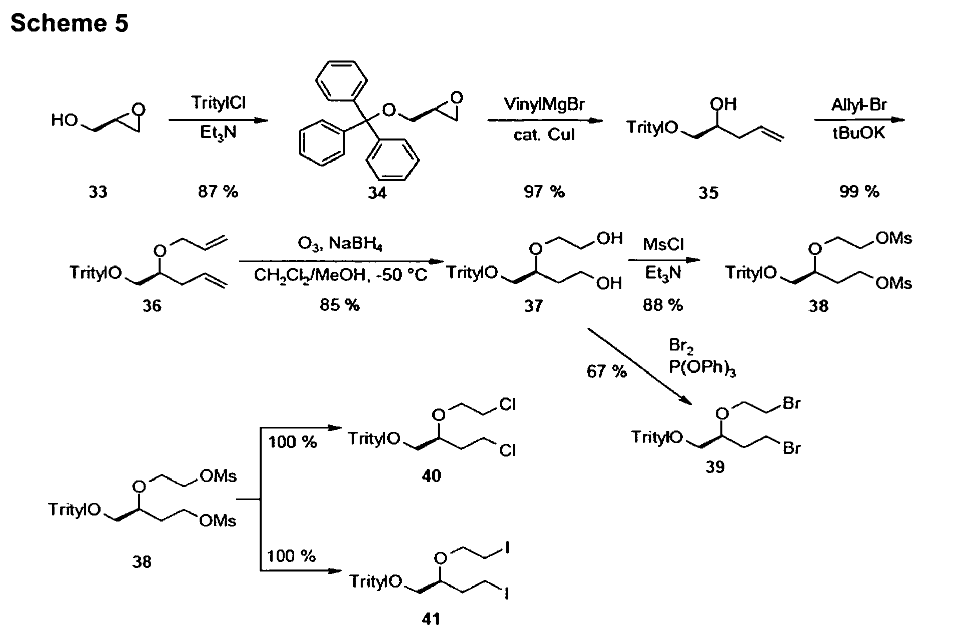

By using R-1-1chloro-2,3-propanediol (98 %ee) as starting material it is possible to obtain trityl-protected R-glycidol with an enantiomeric purity > 98 %ee (Scheme 6).

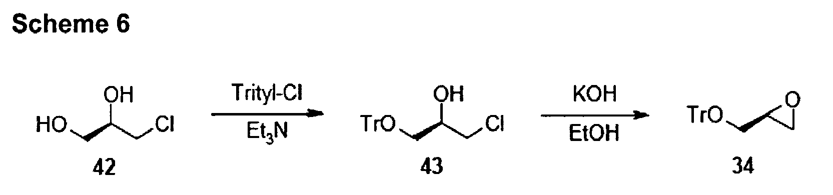

However, the major drawback of this approach is the final macrolactonization (Scheme 7). The best yield obtained for this cyclization is obtained using the bromide with an addition time of 60 h at a concentration of 0.029 M. These long addition times are not acceptable for a technical process. An optimization led to the use of the mesylate, addition times of 6 h at a concentration of 0.029 M gave the desired product in 54 % yield (product was directly crystallized). In addition it has to be taken into account that the iodide and mesylate decompose at room temperature and need to be stored at -5 °C.

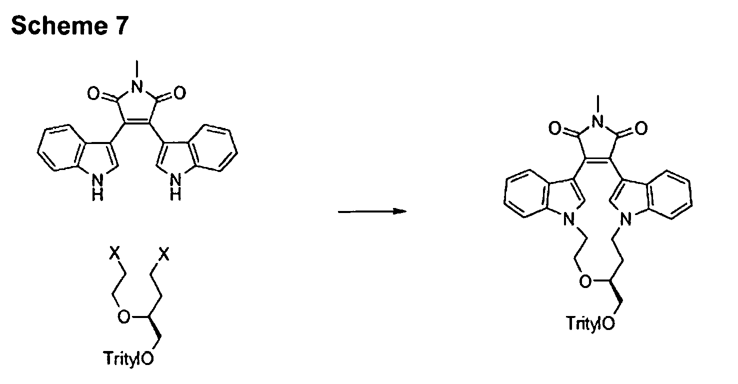

In addition to these problems it is necessary to remove the N-methyl group of the maleimide by a two step hydrolysis/ammonolysis sequence.
Alternatively it was investigated if a consecutive execution of the alkylation by employing a mono-protected spacer yields better results (Scheme 8).

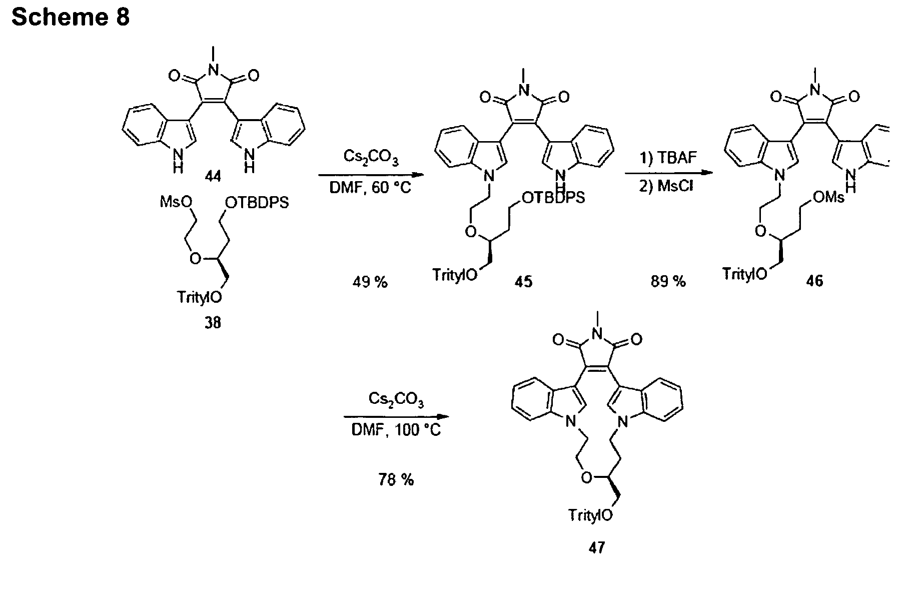

### References

1 EP0657458 B1: Protein Kinase C Inhibitors
2 a) Y. Nishizuka, Annu. Rev. Biochem. 1989, 58, 31-44.
2 b) Y. Nishizuka Science 1992, 258, 607-614.
3 R. M. Bell, D. J. Burns, J. Biol. Chem. 1991, 266, 4661-4664.
4 Inoguchi, Proc. Natl. Acad. Sci. USA 1992, 89, 11059-11065.
5 E. J. Bastyr III, J. Lu, Diabetes (Suppl. 1), 1993, 42, 97A.
6 a) Hsieh, Proc. Natl. Acad. Sci. USA, 1991, 88, 9315-9319
6 b) Hsieh, J. Biol. Chem. 1993, 268, 15118-15126.
7 Murray, J. Biol. Chem. 1993, 268, 15847-15853.
8 a) Tamaoki, Biochem. Biophys. Res. Commun. 1986, 135, 397-402.
8 b) Gross, Biochem. Pharmacol. 1990, 40, 343-350.
9 U. T. Ruegg, G. M. Burgess, Trends Pharmacol. Sci. 1989, 10, 218-220
10 a) Davis, FEBS Lett. 1989, 259, 61-63.
10 b) Twoemy, Biochem. Biophys. Res. Commun. 1990, 171, 1087-1092.
10 c) Toullec, J. Biol. Chem. 1991, 266, 15771-15781.
10 d) Davis, J. Med. Chem. 1992, 35, 994-1001.
10 e) Bit, J. Med. Chem. 1993, 36, 21-29.
11 EP 0410389: Indolocarbazol and its use
12 EP0508792: Anti-tumor and anti-psoriatic agents
13 WO94/07895: Diindole compounds and pharmaceutical compositions containing them.
14 a) McGlynn, J. Cell. Biochem. 1992, 49, 239-250.
14b) N. E. Ward, C. A. O'Brian, Molec. Pharmacol. 1992, 41, 387-392.
15 Toullec, J. Biol. Chem. 1991, 266, 15771-15781.
16 a) Martiny-Baron, J. Biol. Chem. 1993, 268, 9194-9197.
16b) Wilkinson, Biochem. J. 1993, 294, 335-337.
17 M. M. Faul and Ch. A. Krumrich, J. Org. Chem 2001, 66, 2024 -2033.
18 JP1995238044
19 CA2137203
20 US5624949: Protein Kinase Inhibitors
21 JP2004269543:
22 M. R Jirousek,.J. R. Gillig, C. M. Gonzalez, W. F. Heath, J. H. McDonald III., D. A. Neel,C. J. Rito, U. Singh, L. E. Stramm, A. Melikian-Badalian, M. Baevsky, L. M. Ballas, S. E: Hall, L. L. Winneroski, M. M. Faul, J. Med. Chem. 1996, 39, 2664.
23 J. C. Caille, Org. Process. Res. Dev. 2002, 6, 471.
24 W002059342: Methods of preparing S-3-[2-{(methylsulfonyl)oxy}-ethoxy]-4-(triphenylmethoxy)-1-butanol methane sulfonate.
25 a) US5721272: Intermediates and their use to prepare Bisindolylmeleinimides.
25 b) EP0776899: Intermediates and their use to prepare N,N-bridged Biisindolylmeleinimides.
25 c) WO199701880
25 d) EP0776895: Protein Kinase C inhibitors.
25 e) US5710145: Protein Kinase C inhibitors.
25 f) JP1999500149
25 g) US5541347: Synthesis of Bisindolylmaleinimides.
26 EP657411B1: Synthesis of Bisindolylmaleinimides.

### Summary of the invention

The present invention consists in a method of manufacturing ruboxistaurin, comprising the steps of preparation of L-2-deoxyribose 1-cyano-3,4-dibenzoate, followed by deprotection to 1-cyano L-2-deoxyribose, oxidative cleaving to (S)-4-hydroxy-2-(2-hydroxy-ethoxy)-butyronitrile and transformation to the methanesulfonic acid (S)-3-cyano-3-(2-methanesulfonyloxy-ethoxy)-propyl ester, then coupling with 1-substituted-3,4-bis(3-indolyl)maleimide leading to 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-substituted-9(S)-cyano, of formula 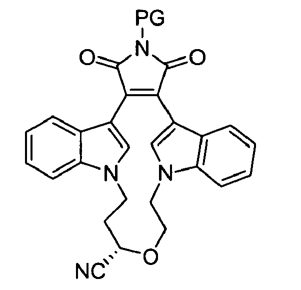 wherein PG is a protective group, which can be an alkyl having 1 to 4 carbons, an alkylaryl or the benzyl or substituted benzyl group, and subsequent reduction of the cyano group to 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-substituted- 9(S)-aminomethyl, of formula 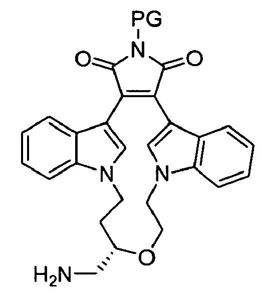 followed by methylation of the amino group and deprotection forming 5,21:12,17-dimetheno-9H-dibenzo[e,k]furo[3,4-h][1,4,13]oxadiazacyclohexadecine-18,20-dione, 6,7,10,11-tetrahydro-9-[(dimethylamino)methyl]-, (S)- (9CI), of formula 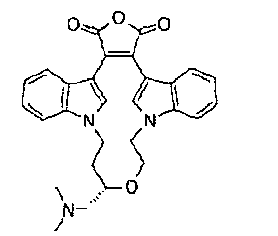 and the final step consisting of imido preparation to form ruboxistaurin.

The reaction steps of the invention are schematically described in the following scheme where ruboxistaurin can be prepared by the mentioned reaction steps. Deoxyribose was used as starting material. D-2-Deoxyribose 1-cyano-3,4-tribenzoate was an intermediate which was necessary to deprotect. Only the 1-β anomer was isolated in solid form. The cis-protected diol was deprotected by treatment of ammonia solution in methanol. Progress of the reaction can easily be monitored by gradual dissolution of the starting material which is not soluble in methanol in contrast to the product, which is soluble. The cleavage of the diol was done with NalO₄. The reduction of the intermediate bis-aldehyde was carried out by addition of NaBH₄ directly. Bis-mesylate was obtained using methanesulfonyl chloride in the presence of triethylamine in dichloromethane at 0 °C. This intermediate was coupled with N-benzyl bisindolylmaleimide in DMF using cesium carbonate as a base. The cyano group was hydrogenated to the primary amine which was subsequently di-alkylated by formic acid. Maleimide was hydrolyzed to anhydride by ethanolic potassium hydroxide. The final steps consist of imide formation. Using D-2-deoxyribose as a starting material for the above-described procedure the enantiomer of ruboxistaurin was received. The unnatural L-2-deoxyribose gives ruboxistaurin.

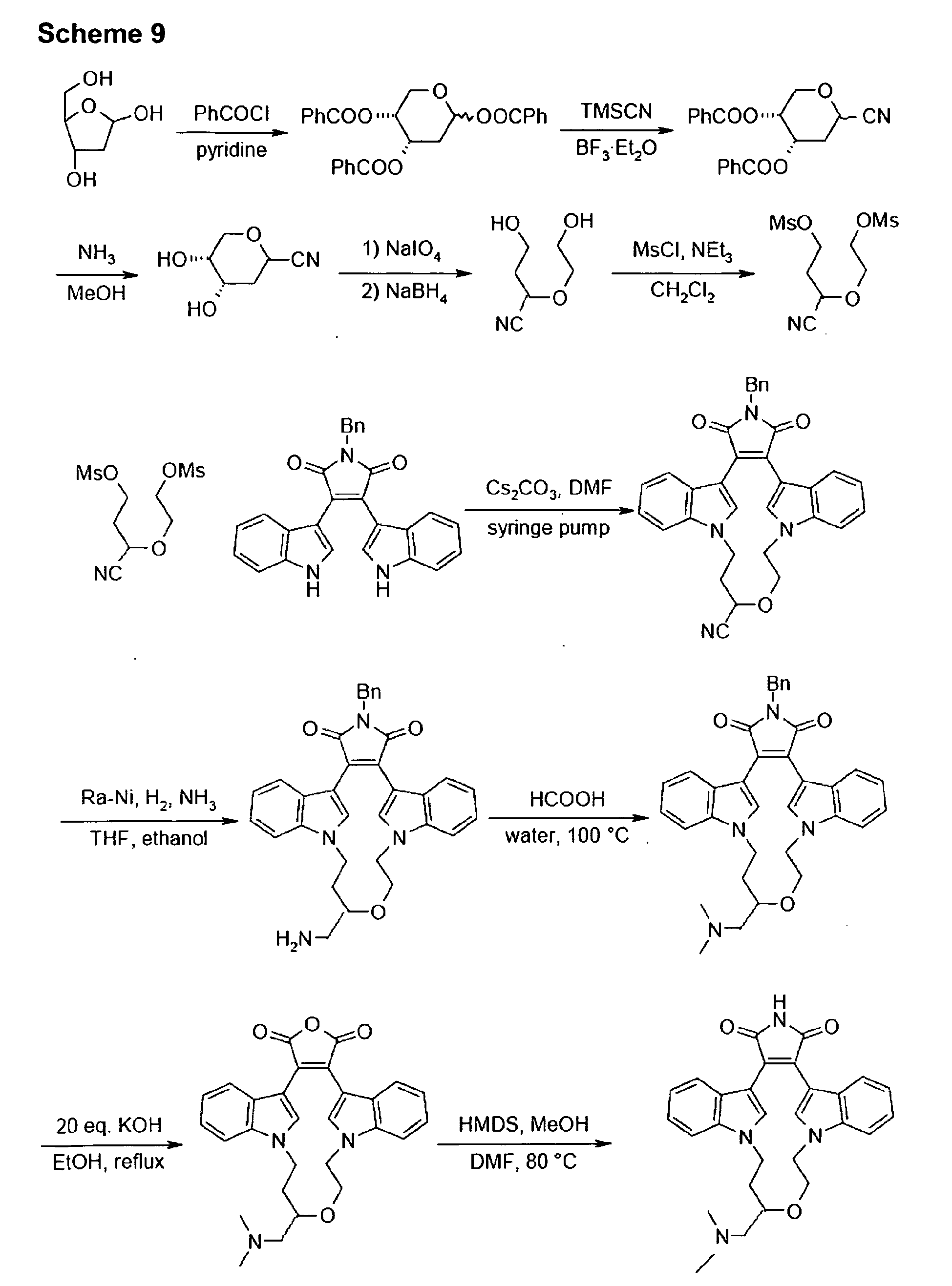

### Detailed description of the invention

Based upon a publication by Togo¹⁸ et al. ribose was used as the starting material. Using the described reaction conditions **12514** was prepared in good yields. The subsequent transformation towards the α- anomer (**12530**) and β-anomer (**12606**) was carried out by using the same conditions as described in the publication by Togo¹⁸. The described chromatographic separation of the anomers was successfully performed on small scale. It was observed that in methanol a precipitate formed and a detailed analysis revealed that by treating the mixture of anomers with methanol the β-anomer can selectively be isolated by precipitation.

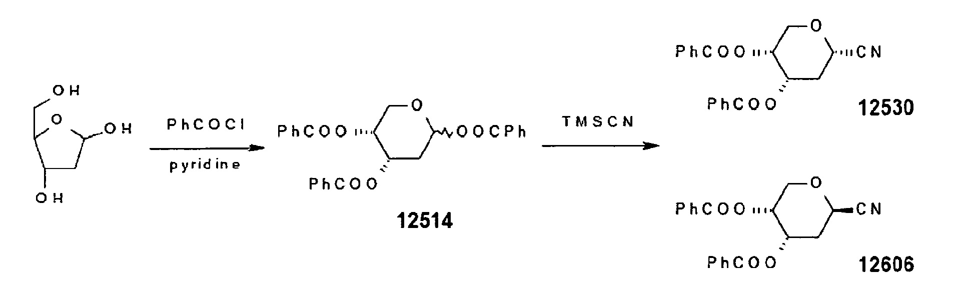

For the subsequent step of the attempted sequence it was necessary to deprotect the cis-diol which was done by using a solution of ammonia in methanol. Progress of the reaction can easily be monitored by gradual dissolution of the starting material which is not soluble in methanol to **1608,** which is soluble.

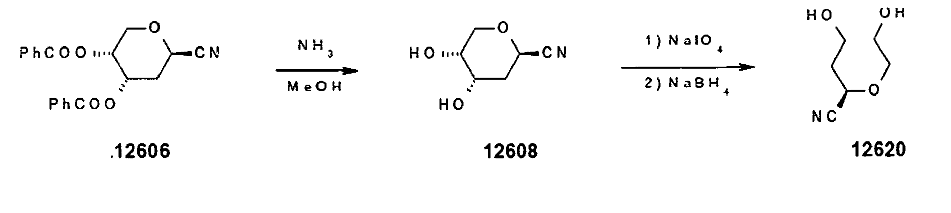

The cleavage of the diol was carefully investigated and it was observed that the reaction with NalO₄ is very slow and that the reaction mixture should preferentially be stirred at RT in order to achieve fast conversion. The reduction of the intermediate bis-aldehyde was carried out by addition of NaBH₄ directly into the reaction mixture. The reaction can be monitored by TLC (staining with KMnO₄ or phenylhydrazine).

The activation of **12608** towards the bis-mesylate was performed by methanesulfonyl chloride in the presence of triethylamine in dichloromethane at 0 °C. The product was purified by extraction and was used without further purifications in the next steps. By storing the product at 0 °C no decomposition within several days was detected.

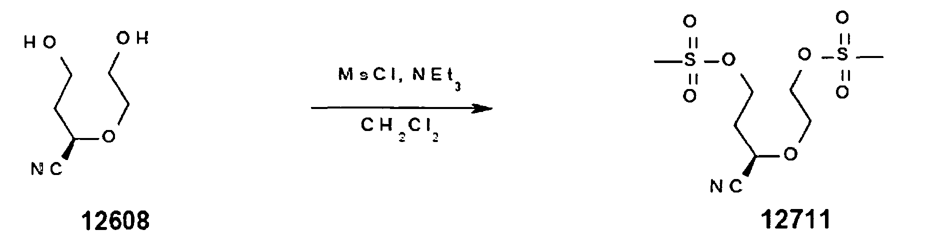

Having synthesized **12711,** it was attempted to prepare the PMB-protected precursor **12723.** Therefore the same conditions as described in literature ( M. M. Faul at all. J. Org. Chem. 1998, 63, 1961-1973.) were used.
Caesium carbonate was used as base in DMF as solvent and neither the concentration nor the addition time was changed. This resulted in the isolation of the desired product in 47 % yield.

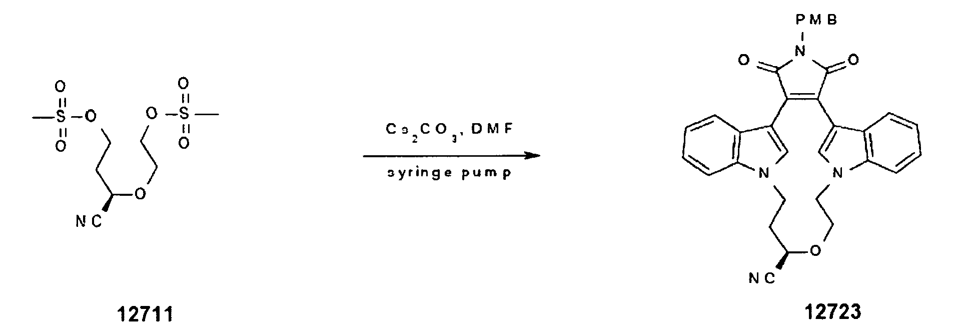

Encouraged by these results it was attempted to use unprotected 3,4-Bis(3-indolyl)maleimide as the coupling partner. Even though the same reaction conditions were use as previously successfully used it was not possible to detect any formation of product. We observed only detectable product (or an isomer of it) is displayed in the following scheme 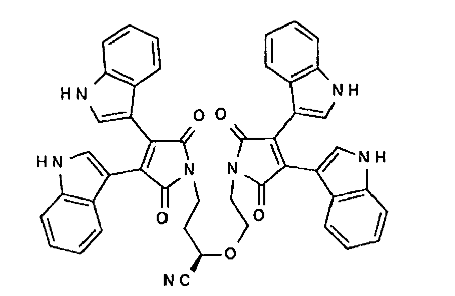 and even this was only detected at the beginning (∼1/6 of **12711** was added) of the reaction. After completion of addition even this product was no longer detectable by LC/MS, which is probably due to oligomerization or further reactions of this intermediate with **12711.**

Based on these results the coupling with benzyl protected **12689** was investigated using the same conditions as previously described.

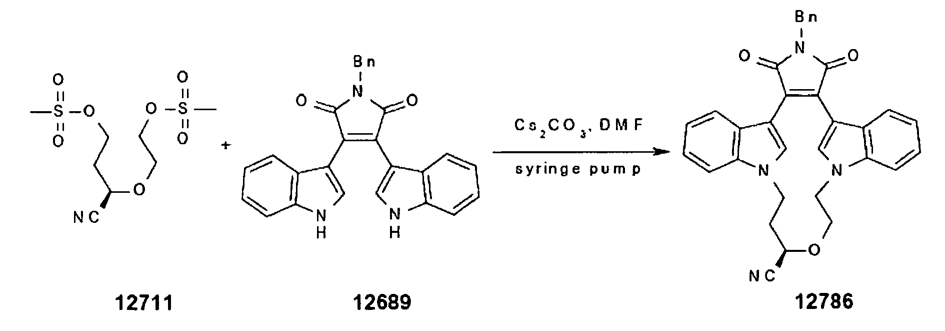

The first reaction was carried out on a 100 mg scale. The solution of the mesylate was added within -6 h and the product was isolated in 61 % yield after chromatography. During the upscale the solution of the mesylate was accidentally added to fast (-50 % within 2 h, the rest within 4 h). **12786** was isolated in 59 % yield. Based on these results it has to be expected that a further upscale of this step can be made and that overly extended additions times are not necessarily required for the macrocyclization.
Interestingly it was not possible to detect the acyclic intermediate. The same result is described by lit (M. M. Faul et al J. Org. Chem. 1998, 63, 1961-1973.)

They described that the second alkylation is extremely rapid compared to the first one. This is probably due to the geometry of the molecule with a pre-orientation of the second indole.

Reduction of nitrile was done by hydrogen.

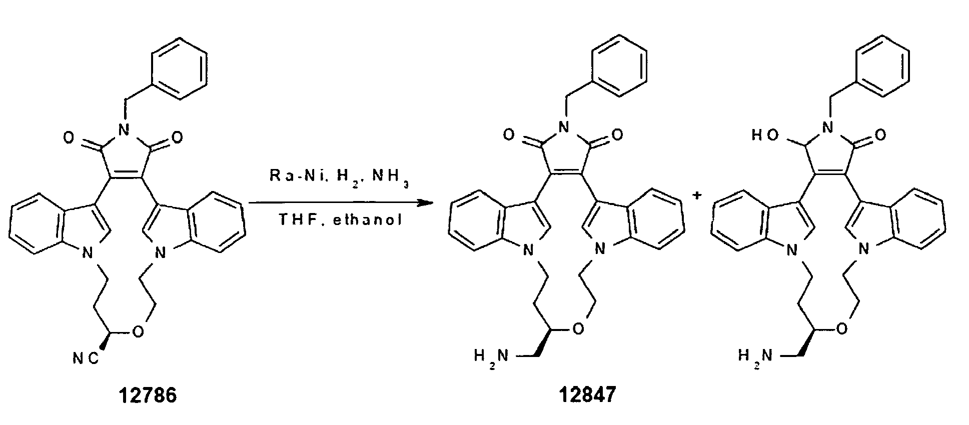

The first approach was using Raney nickel in ethanol in the presence of ammonia and hydrogen. The formation of product was observed, even though a possible side product, presumably a hydroxyl-lactone, was also formed based on LC/MS results. During a following experiment the formation of this side product was almost completely suppressed which can be a result of a large amount of catalyst used.

The subsequent bis-methylation of the primary amine **12847** was carried out by using typical conditions such as aq. formaldehyde/Na(AcO)₃BH/DCE, Mel/Na₂CO₃/DMSO, aq. formaldehyde/hydrogen/Pt. All the reactions were monitored by LC/MS and in all cases it was not possible to detect any product.
However, by applying Eschweiler-Clark conditions, e.g. urotropine and formic acid in water at ∼100 °C the product was formed and the desired product was isolated by chromatography in almost quantitative yield.

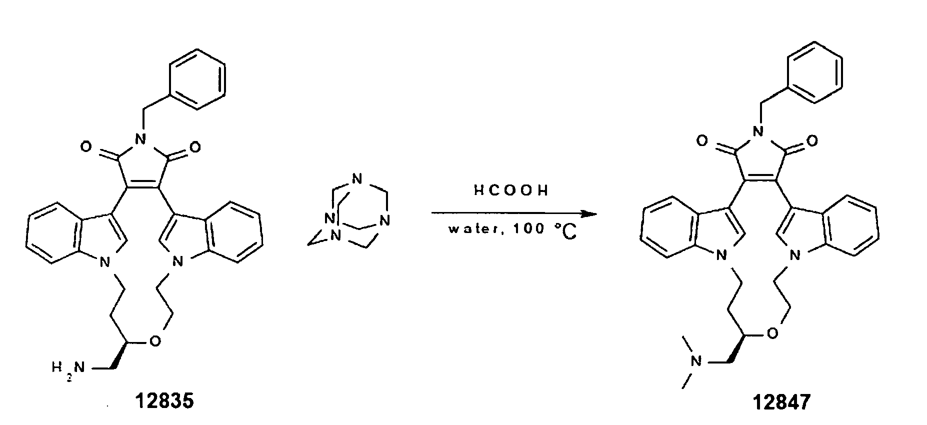

The hydrolysis of **12847** towards the anhydride **12886** with ethanolic potassium hydroxide required an acidic work-up, which is mandatory. If the acidic work-up is not performed, a mixture of the desired anhydride and the intermediate acid-amide is observed.

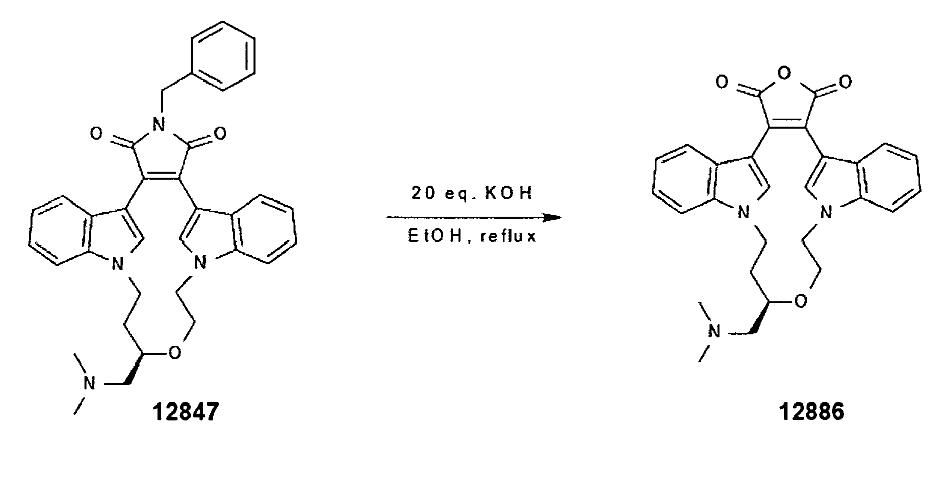

The final step of this route is the treatment of **12886** with HMDS in the presence of methanol in DMF. The reaction proceeds overnight and the product can be obtained after acidic work-up in pure form. Interestingly it is possible to extract **12899** with 0.5 N HCl into the aqueous phase for purification. By neutralizing the solution with 1 N NaOH it was important to keep the pH between 7-9 in order to overcome two many extractions with diethyl ether or dichloromethane to ensure good yields. This is due to the solubility of **12899** under basic conditions.

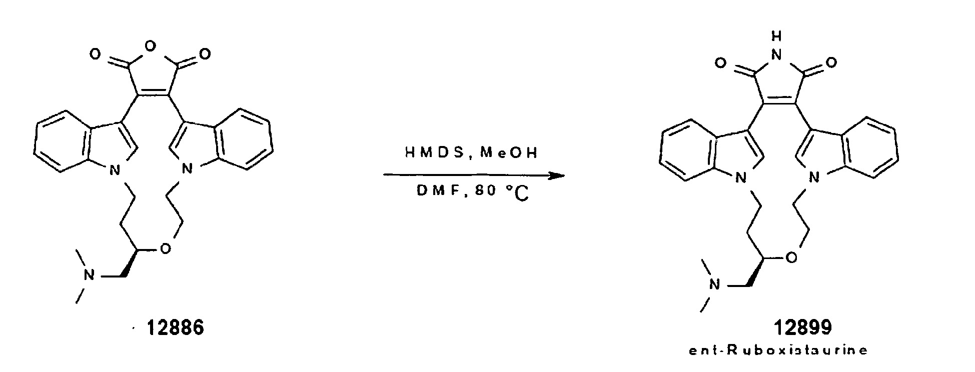

All the synthetic steps together are described in following scheme:

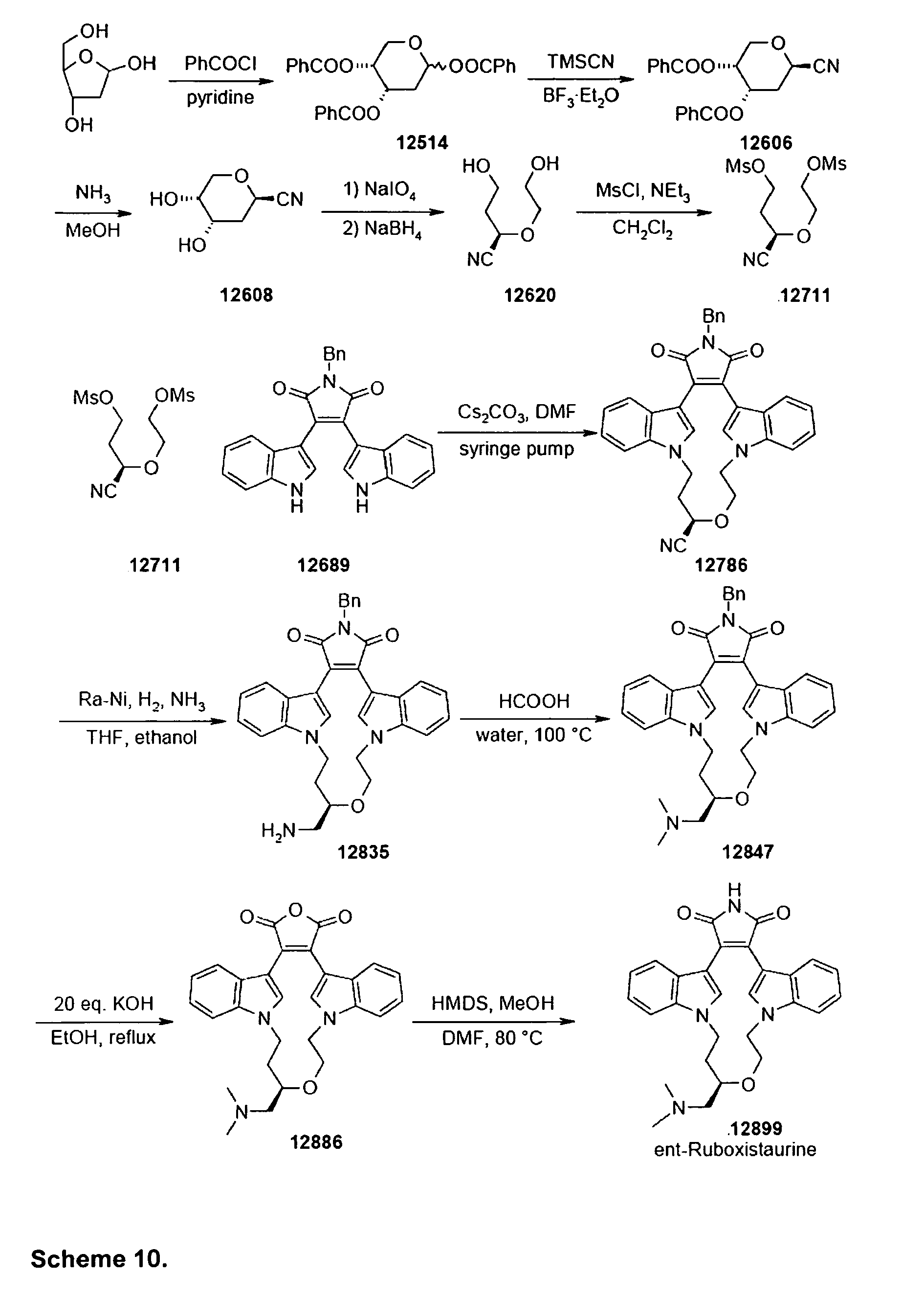

### Experimental Examples

### Example 1

### D-2-Deoxyribose 1,3,4-tribenzoate (12514)

To a solution of 22.0 g (164 mmol) D-deoxy-ribose in 340 mL dichloromethane were added at 0 °C 73.0 mL (629 mmol) benzoylchloride. The reaction mixture was stirred for 3 h at 0 °C. After completion of the reaction sat. NaHCO₃ was slowly added until no further gas development was observed and the product was extracted with chloroform. The combined organic layers were dried over sodium sulphate and the solvent was removed. The residue was dissolved in 100 mL dichloromethane. 1000 mL diethyl ether were added and the mixture was stirred overnight. The precipitate was collected and dried in vacuo to yield 16.1 g **12514.** The solvent of the filtrate was removed and the residue was taken up in diethyl ether. The mixture was stirred at 0 °C. The precipitate was collected and dried in vacuo to yield 6.86 g **12514.** The solvent of the filtrate was removed and the residue was taken up in diethyl ether. The mixture was stirred at -10 °C. The precipitate was collected and dried in vacuo to yield 14.3 g **12514.** The crops were combined to yield 37.2 g **12514.**
δ ¹³C NMR (CDCl₃) : 30.75 (CH2), 63.80 (CH2), 66.72 (CH), 68.38 (CH), 92,91 (CH) 128.80, 128.90, 130.21, 134.75, 166.02 ( 3x benzoyl)

### Example 2

### D-2-Deoxyribose 1- β-cyano-3,4-dibenzoate (12606)

To a solution of 37.2 g (83.3 mmol) **12514** in 340 mL dichloromethane were added at 0 °C 13.4 mL (99.9 mmol) TMSCN. The reaction mixture was stirred for 5 min at 0 °C. 30.6 mL (249 mmol BF₃·Et₂O were slowly added. The resulting reaction mixture was stirred for 1 h at 0 °C and the reaction was monitored by HPLC. After completion of the reaction sat NaHCO₃ was added and the mixture was stirred for 10 min at RT. The product was extracted with dichloromethane. The combined organic layers were dried over sodium sulphate and the solvent was removed. The residue was taken up in methanol and stirred overnight. The precipitate was collected and dried in vacuo to yield 12.9 g **12606.**

### Example 3

### 1- β-Cyano D-2-deoxyribose (12608)

A suspension of 12.9 g (36.7 mmol) **12606** in 100 mL 15% NH₃ in methanol was stirred overnight and the reaction was monitored by TLC (ethyl acetate, UV, KMnO₄). The solvent was removed and the product purified by chromatography (500 g silica gel, ethyl acetate until impurity removed, then ethyl acetate/ethanol 1:1) to yield 4.97 g of an oil which crystallized upon standing **12608**
δ ¹³C NMR (DMSO-d6): 33.65 (CH2), 62.21 (CH), 65.12 (CH), 66.66 (CH), 67.60 (CH2), 119.00 (CN)

### Example 4

### (R)-4-Hydroxy-2-(2-hydroxy-ethoxy)-butyronitrile (12620)

To a solution of 2.69 g (18.7 mmol) **12608** in 150 mL methanol and 50 mL water were added at 0 °C 4.42 g (20.6 mmol) NalO₄. The reaction was stirred 1 h at 0 °C and monitored by TLC (ethyl acetate, KMnO₄, phenylhydrazine). The reaction stirred at RT and monitored by TLC. After completion of the reaction 1.56 g (41.3 mmol) NaBH₄ were added. The reaction was stirred at RT and monitored by TLC. After completion of the reaction 2 N HCl was added until a brownish solution was obtained. The solvent was removed and the product was purified by chromatography (200 g silica gel, ethyl acetate until impurities removed then ethyl acetate/ethanol 3:1). A further purification by chromatography (200 g silica gel, ethyl acetate until impurities removed then ethyl acetate/ethanol 3:1) yielded 1.39 g **12620** as colourless oil.
δ ¹H NMR (DMSO-d6): 1.92( m ,2H), 3.5-3.7 (m,6H), 4.57 (t, 1H)

### Example 5

### Methanesulfonic acid (R)-3-cyano-3-(2-methanesulfonyloxy-ethoxy)-propyl ester (12711)

To a solution of 1.29 g (8.88 mmol) **12620** and 2.42 g (23.9 mmol) triethylamine in 100 mL dichloromethane were added at 0 °C 3.05 g (26.6 mmol) methanesulfonyl chloride. The reaction mixture was stirred at 0 °C and the reaction was monitored by TLC (ethyl acetate, KMnO₄, UV). After completion (-2 h) of the reaction water was added. The organic layer was washed with 2 N HCl, 0.5 N NaOH and water. The organic layer was dried over sodium sulphate and the solvent was removed to yield 2.41 g 12711 which was used without further purification.
δ ¹³C NMR (CDCl₃) : 33.69 (CH2), 37.90 (CH3), 38.20 (CH3), 64.38 (CH2), 65.67 (CH), 67.86 (CH2), 68.96 (CH2), 117.40 (CN)

### Example 6

### 9H,18H-5,21:12,17-Dimethenodibenzo[e,k]pyrrolo[3,4-h][1,4,13] oxadiazacyclohexadecine-18,20(19H)-dione, 6,7,10,11-tetrahydro-19-(phenylmethyl)-9-cyano-,(9R)- (9CI) (12786)

To a solution of 1.15 g (2.76 mmol) **12689** and 1.86 g (5.80 mmol) Cs2CO3 in 80 mL DMF was slowly added at 100 °C a solution of 1.00 g (3.31 mmol) **12711** in 20 mL DMF (-8 mL within 90 min, then -12 mL within 6 h). The solution was stirred 8 h at 100 °C. After cooling to RT the product was extracted with ethyl acetate. The combined organic layers were washed with brine and dried over sodium sulphate. The solvent was removed and the product was purified by chromatography (30 g silica gel, ethyl acetate/heptane 3:1, UV, KMn04) to yield 860 mg **12786** as red solid. MS(ES): M+1 = 527
δ ¹³C NMR (CDCl₃) characteristic signals: 33.76 (CH2), 42.12 (CH2), 43.61 (CH2), 45.82 (CH2), 69.48 (CH), 69.52 (CH2), 116.90 (CN), 171.36 (CO), 171.39 (CO)

### Example 7

### 9H,18H-5,21:12,17-Dimethenodibenzo[e,k]pyrrolo[3,4-h][1,4,13] oxadiazacyclohexadecine-18,20(19H)-dione, 6,7,10,11-tetrahydro-19-(phenylmethyl)-9-aminomethyl-,(9R)- (9CI) (12835)

A mixture of 250 mg (474 µmol) **12786** and 120 Raney nickel in 25 mL ammonia in ethanol and 25 mL THF was stirred under 50 bar hydrogen at 50 °C. The reaction was monitored by LC/MS. After completion of the reaction the catalyst was removed by filtration over Celite. The solvent was removed and the product was purified by chromatography (60 g silica gel, ethyl acetate/ethanol/triethylamine 20:10:3, UV) to yield 44.8 mg **12835.**
MS(ES): M+1 = 531

### Example 8

### 9H,18H-5,21:12,17-Dimethenodibenzo[e,k]pyrrolo[3,4-h][1,4,13] oxadiazacyclohexadecine-18,20(19H)-dione, 6,7,10,11-tetrahydro-19-(phenylmethyl)-9-dimethylaminomethyl-,(9R)- (9CI) (12847)

To a solution of 44.8 mg (82.9 µmol) **12835** and 17.4 mg (124.3 µmol) urotropine in 1 mL water were added 57.3 mg (124.3 µmol) formic acid. The reaction mixture was stirred at RT for 15 min and then heated to 100 °C. The reaction was monitored by HPLC. After completion of the reaction the reaction mixture was allowed to cool to RT and THF was added until a homogenous solution was obtained. The product was purified by chromatography (15 g silica gel, ethyl acetate/ethanol 1:1 to 1:2) to yield 44.0 mg **12847.**
MS(ES): M+1 = 559

### Example 9

### 5,21:12,17-Dimetheno-9H-dibenzo[e,k]furo[3,4-h][1,4,13]oxadiazacyclohexadecine-18,20-dione, 6,7,10,11-tetrahydro-9-[(dimethylamino)methyl]-, (R)- (9CI) (12886)

To a suspension of 44.0 mg (78.8 µmol) **12847** in 3 mL ethanol were added 110 mg (414.5 µmol) KOH. The red solution turned yellow and the reaction was monitored by HPLC (IMPORTANT: for sampling a drop of the reaction mixture was added to a 0.5 N HCl/acetonitrile solution. This solution was kept at RT for 5 min before measurement). After completion of the reaction the solution was allowed to cool to RT, -5 mL 0.5 N HCl were added and the reaction mixture was stirred at RT for 15 min. The product was extracted with dichloromethane. The combined organic layers were dried over sodium sulphate and the solvent was removed. The product was used without further purification.
MS(ES): M+1 = 470

### Example10

### ent-Ruboxistaurine (12899)

To a solution of 44.0 mg (78.8 µmol) **12886** in 1 mL DMF were added at RT 144 mg (82.9 µmol) HMDS and 13.2 mg (415 µmol) methanol. The reaction mixture was stirred overnight at 85 °C and the reaction was monitored by HPLC. After completion of the reaction the mixture was allowed to cool to RT and the solvent was removed. The residue was taken up in -10 mL 0.5 N HCl and the solution was stirred for 10 min at RT. The aqueous layer was washed with ethyl acetate. 10 mL 1: NaOH was added and the product was extracted with ethyl acetate. The combined organic layers were dried over sodium sulphate and the solvent was removed. The residue was dissolved in 10 mL 0.5 N HCl and the aqueous layer was washed with diethyl ether. The aqueous layer was neutralized with 1 N NaOH and the product was extracted with diethyl ether. The combined organic layers was dried over sodium sulphate and the solvent was removed to yield 16.8 mg **12899.**
MS(ES): M+1 = 469

## Claims

1. Method of manufacturing ruboxistaurin, comprising the steps of preparation of L-2-deoxyribose 1-cyano-3,4-dibenzoate, followed by deprotection to 1-cyano L-2-deoxyribose, oxidative cleaving to (S)-4-hydroxy-2-(2-hydroxy-ethoxy)-butyronitrile and transformation to the methanesulfonic acid (S)-3-cyano-3-(2-methanesulfonyloxyethoxy)-propyl ester, then coupling with 1-substituted-3,4-bis(3-indolyl)maleimide leading to 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-substituted-9(S)-cyano, of formula 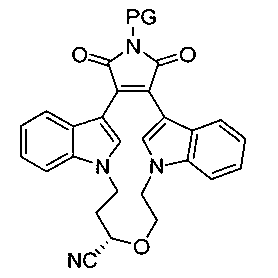 wherein PG is a protective group, which can be an alkyl having 1 to 4 carbons, an alkylaryl or the benzyl or substituted benzyl group,
and subsequent reduction of the cyano group to 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-substituted- 9(S)-aminomethyl, of formula 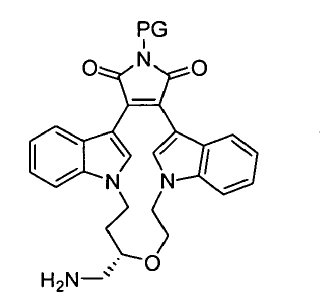 followed by methylation of the amino group and deprotection forming 5,21:12,17-dimetheno-9H-dibenzo[e,k]furo[3,4-h][1,4,13]oxadiazacyclohexadecine-18,20-dione, 6,7,10,11-tetrahydro-9-[(dimethylamino)methyl]-, (S)- (9CI), of formula 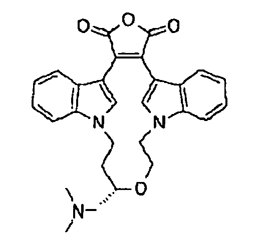 and the final step consisting of imido preparation to form ruboxistaurin.

2. 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-(phenylmethyl)-9(S)-cyano as an intermediate for ruboxistaurin.

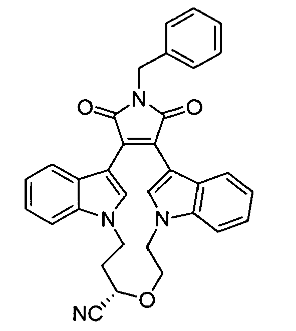

3. 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-(phenylmethyl)-9(S)-aminomethyl as an intermediate for ruboxistaurin.

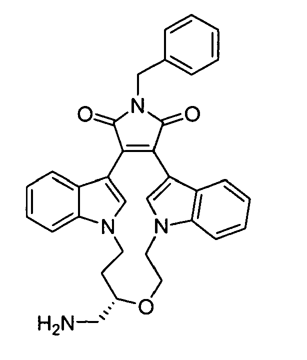

4. Use of 1,2,3-triacyl -L- 2-deoxyribose as a starting material to prepare (S)-3-cyano-3-(2-methanesulfonyloxy-ethoxy)) propyl ester as an intermediate for ruboxistaurin.

5. Use of 1,2,3-triacyl -L- 2-deoxyribose as a starting material to prepare (S)-3-cyano-3-(2-methanesulfonyloxy-ethoxy)) propyl ester as an intermediate for ruboxistaurin.

6. Method of manufacturing a ruboxistaurin enantiomer, comprising the steps of the preparation of D-2-deoxyribose 1-cyano-3,4-dibenzoate, followed by deprotection to 1-cyano D-2-deoxyribose, oxidative cleaving to (R)-4-hydroxy-2-(2-hydroxy-ethoxy)-butyronitrile and transformation to the methanesulfonic acid (R)-3-cyano-3-(2-methanesulfonyloxy-ethoxy)-propyl ester, then coupling with 1-substituted-3,4-bis(3-indolyl)maleimide leading to 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-substituted-9(R)-cyano, of formula 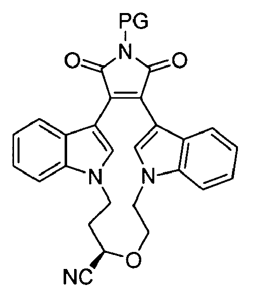 wherein PG is a protective group, which can be an alkyl having 1 to 4 carbons, an alkylaryl or the benzyl or substituted benzyl group,
and subsequent reduction of the cyano group to 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-substituted- 9(R)-aminomethyl, of formula 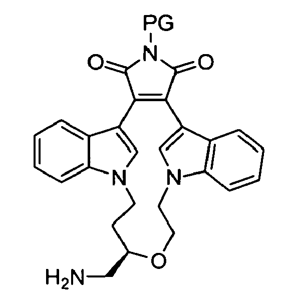 followed by methylation of the amino group and deprotection forming 5,21:12,17-dimetheno-9H-dibenzo[e,k]furo[3,4-h][1,4,13]oxadiazacyclohexadecine-18,20-dione, 6,7,10,11-tetrahydro-9-[(dimethylamino)methyl]-, (R)- (9CI), of formula 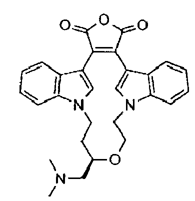 and the final step consisting of imido preparation to form a ruboxistaurin enantiomer.

7. 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-(phenylmethyl)-9(R)-cyano as an intermediate for ruboxistaurin enantiomer preparation.

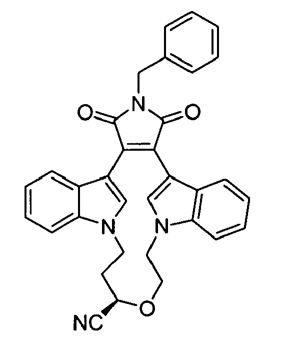

8. 9H,18H-5,21:12,17-dimethenodibenzo[e,k]pyrrolo[3,4-h][1,4,13] oxadiazacyclohexadecine-18,20(19H)-dione, 6,7,10,11-tetrahydro-19-(phenylmethyl)-9(R)-aminomethyl as an intermediate for ruboxistaurin enantiomer preparation.

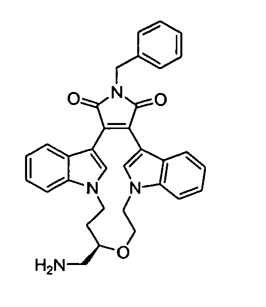

9. Use of 1,2,3-triacyl -D- 2-deoxyribose as a starting material to prepare (R)-3-cyano-3-(2-methanesulfonyloxy-ethoxy)) propyl ester as an intermediate for ruboxistaurin enantiomer

10. Use of (R)-4-Hydroxy-2-(2-hydroxy-ethoxy)-butyronitrile as an intermediate for ruboxistaurin enantiomer preparation.
